# FASCICULE DE BREVET EUROPEEN

(11) **EP 0 648 204 B1**
(45) Date de publication et mention de la délivrance du brevet: **18.11.1998**
(21) Numéro de dépôt: 94913635.2
(22) Date de dépôt: 11.04.1994
(51) Int. Cl.: C07C 271/16, A61K 7/025, A61K 7/031, A61K 7/032, A61K 7/06, A61K 7/08, A61K 7/09, A61K 7/13, A61K 7/32, A61K 7/42, C07C 233/18

(54) **UTILISATION EN COSMETIQUE DE DERIVES LIPOPHILES DES AMINO DEOXYALDITOLS, COMPOSITIONS COSMETIQUES LES CONTENANT, ET NOUVEAUX CARBAMATES D'ALKYLE**
KOSMETISCHE ANWENDUNG VON LIPOPHILES DERIVATES VON AMINODEOXYALDITOLES, DIESE ENTHALTENDE KOSMETISCHE ZUSAMMENSETZUNGEN UND ALKYLCARBAMATES
AMINO DEOXYALDITOL LIPOPHILIC DERIVATIVES FOR USE IN COSMETICS, COSMETIC COMPOSITIONS CONTAINING SAME AND NOVEL ALKYL CARBAMATES

(30) Priorité: 15.04.1993 FR 9304444
(43) Date de publication de la demande: 19.04.1995
(73) Titulaire: L'OREAL, 75008 Paris (FR)
(72) Inventeur: MAHIEU, Claude, F-75017 Paris (FR); SEMERIA, Didier, F-77181 Courtry (FR); CAUWET, Danièle, F-75011 Paris (FR); VANLERBERGHE, Guy, Villevaudé F-77410 Claye-Souilly (FR)
(74) Mandataire: Casalonga, Axel
(86) Numéro de dépôt international: FR9400402
(87) Numéro de publication internationale: WO9424097

(56) Documents cités:
- EP-A- 0 220 676
- EP-A- 0 285 768
- WO-A-92/05764
- WO-A-92/13059
- US-A- 2 808 402

## Description

La présente invention a pour objet l'utilisation en cosmétique de dérivés lipophiles d'amino déoxyalditols, et les compositions cosmétiques contenant un ou plusieurs de ces dérivés.

Il est connu que les poils des mammifères contiennent un certain nombre de composés lipidiques dont la structure et la répartition ne sont pas encore parfaitement définies. A côté des lipides apolaires sécrétés par les glandes sébacées et longtemps considérés comme étant les seuls présents sur les poils, la présence d'autres lipides polaires a été mise en évidence; parmi ceux-ci le sulfate de cholestérol, des acides gras et des alcools gras. Ces composés polaires ont longtemps échappé aux investigations des chercheurs, probablement du fait de leur très faible solubilité dans les solvants d'extraction usuels à base de chloroforme, méthanol et hexane. Leur extraction est si difficile que certains soupçonnent même (pour certains d'entre eux), leur liaison covalente avec les surfaces des cellules (P.W. WERTZ. Lipids 23 n° 9 (1988) 878-881).

Chez L'homme, les lavages fréquents, l'usure, les agressions climatiques et certains traitements cosmétiques auxquels sont soumis les cheveux, les cils, la barbe, sont responsables de la diminution des propriétés esthétiques des poils en général.

Il est donc apparu nécessaire dans le domaine cosmétique et en particulier pour des compositions de traitement des cheveux de réparer les dommages subis par un apport de produits lipidiques polaires qui, du fait de leur très faible solubilité dans l'eau, ne seront pas éliminés lors des lavages, d'autant plus que leur forte polarité permettra leur association à la structure même du cheveu, ce qui ne peut être le cas avec les composés peu ou pas polaires de type cireux souvent préconisés pour améliorer l'état du cheveu.

Certains composés obtenus par extraction à partir de tissus animaux ou végétaux ont déjà été préconisés pour ce type d'application. En particulier des produits tels les céramides ont été décrits dans le document EP-A-278 505. Cependant, la difficulté à les obtenir en quantité industrielle à un taux de pureté suffisant rend l'utilisation de ces composés peu attractive.

Le document WO-92/05764 décrit des compositions pour shampooing contenant au moins 1 % en poids d'un agent tensio-actif choisi parmi les amides d'acides gras polyhydroxylés répondant à la formule : 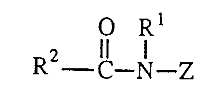 dans laquelle R¹ représente l'hydrogène, un radical hydrocarbyle en C₁-C₄, le 2-hydroxyéthyle, le 2-hydroxypropyle, ou leurs mélanges, et de préférence le radical méthyle; R² est un reste hydrocarbyle en C₅-C₃₁, de préférence un alkyle ou alcényle à chaîne droite en C₉-C₁₇, et tout particulièrement un alkyle ou alcènyle à chaîne droite en C₁₁-C₁₅, ou leurs mélanges ; et Z est un reste polyhydroxycarbyle ayant une chaîne linéaire hydrocarbyle avec au moins 3 hydroxyles liés à la chaîne, ou un dérivé alcoxylé (de préférence éthoxylé ou propoxylé) de celui-ci. Les amides d'acides gras polyhydroxylés, utilisés comme agents tensio-actifs dans les compositions pour shampooing du document WO-92/05764, du fait de leurs propriétés détergentes, doivent avoir une solubilité acceptable dans l'eau, et par conséquent il est recommandé d'utiliser des amides d'acides gras polyhydroxylés ayant des substituants en C₁₂-C₁₈, qui fournissent des matériaux ayant le meilleur compromis entre la facilité de fabrication, la solubilité dans l'eau et l'activité détergente.

Le document WO-92/13059 décrit une plaquette de savon ayant une tendance réduite à la formation de craquelures qui comprend au moins environ 1 % en poids d'un agent tensio-actif amide d'acide gras polyhydroxylé, en particulier un C₁₂-C₁₈ alkyl N-méthyl glucamide.

Le document EP-A-0 285 768 décrit un système tensio-actif comprenant un agent épaississant qui est un N-polyhydroxyalkylamide d'acide gras de formule : 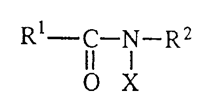 où R¹ est un groupe alkyle en C₁-C₁₇, R² est H ou un groupe alkyle en C₁-C₁₈ et X est un groupe polyhydroxyalkyle en C₄-C₇.

Le document EP-A-0 220 676 décrit un agent tensio-actif de formule : 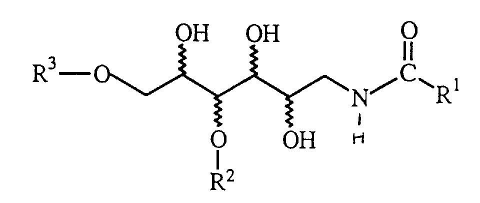 où R¹ est un groupe alkyle en C₇-C₂₁ et R² et R³ représentent H ou un groupe mono ou oligo saccharide lié à un reste α ou β glucoside.

Il est donc apparu nécessaire de rechercher des composés synthétiques parfaitement définis qui soient solides aux températures d'utilisation afin de restructurer les poils sans leur apporter un aspect huileux indésirables et qui de plus soient insolubles dans l'eau seule mais peuvent être déposés sur les cheveux à partir d'une formule essentiellement aqueuse. Ces composés devront apporter après dépôt d'excellentes propriétés au cheveu telles que démêlage, brillance, nervosité et toucher agréable.

La demanderesse a trouvé que des composés répondant particulièrement bien à l'ensemble des critères ci-dessus étaient des dérivés lipophiles des amino déoxyalditols et plus particulièrement des composés non ioniques de type polyols linéaires associés à une longue chaîne grasse, les deux séquences de ces composés amphiphiles étant liées par des fonctions elles-mêmes fortement polaires, de type amide ou carbamate.

De plus, ces composés se sont avérés intéressants dans la formulation de compositions pour l'hygiène corporelle et bucco-dentaire.

Un objet de la présente invention est par conséquent l'utilisation des dérivés ci-dessus dans des compositions cosmétiques.

L'invention a également pour objet les compositions cosmétiques contenant de tels composés, ainsi que leur application pour le traitement des matières kératiniques telles que la peau ou les cheveux, et l'hygiène bucco-dentaire.

L'invention a aussi pour objet de nouveaux dérivés lipophiles d'amino déoxyalditols de type carbamate utilisables dans les compositions selon l'invention.

D'autres objets de l'invention apparaîtront à la lecture de la description et des exemples qui suivent.

Les dérivés lipophiles d'amino déoxyalditols utilisés conformément à l'invention dans des compositions cosmétiques, en particulier pour le traitement de la peau, des cheveux ou l'hygiène bucco-dentaire, sont caractérisés par le fait qu'ils répondent à la formule générale : 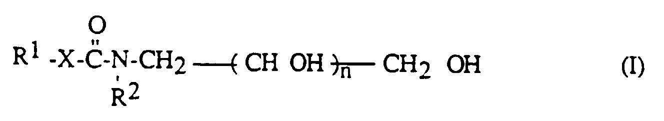
dans laquelle R¹ est un radical aliphatique linéaire saturé en C₁₄-C₄₀;
R² est un atome d'hydrogène ou un radical alkyle linéaire en C₁-C₆;
X est un atome d'oxygène ou un radical méthylène; et
n est un entier de 1 à 5, sous réserve que lorsque X est un radical méthylène, R¹ est un radical aliphatique linéaire saturé en C₁₉-C₃₉.

Certains des composés répondant à la formule générale (I) ci-dessus sont connus. En particulier, US-A-1 985 424 et 2 703 798 décrivent des amides répondant à cette formule générale. Toutefois les seuls composés décrits correspondent à des chaînes lipophiles courtes ou moyennes limitées à 18 atomes de carbone.

US-A-2 040 997 décrit des carbamates répondant à la formule (I) ci-dessus. Ces carbamates répondent à la formule générale 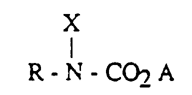 dans laquelle R est un radical aliphatique à substitutions polyhydroxy, X est un atome d'hydrogène ou un radical alkyle et A est un radical hydrocarboné aliphatique ayant 8 ou plus atomes de carbone. De préférence A représente un groupe alkyle à chaîne droite ayant 10 à 20 atomes de carbone. Les carbamates utilisés comme intermédiaires de synthèse ne sont illustrés que par le seul composé dodécyle.

Dans la formule (I) ci-dessus, et lorsque X représente un atome d'oxygène, R¹ représente un radical aliphatique linéaire saturé en C₁₄-C₄₀, de préférence en C₁₄-C₃₂ et mieux encore en C₁₆-C₂₂. Lorsque X représente un radical méthylène, R¹ représente un radical aliphatique linéaire saturé en C₁₉-C₃₉, de préférence C₂₁-C₂₉ ; R² représente un atome d'hydrogène ou un radical alkyle linéaire de C₁ à C₅, de préférence méthyle; et n est un nombre entier de 1 à 5 et de préférence égal à 4.

Parmi, les dérivés de formule (I) recommandés on peut citer le 1-[docosanoyl-méthyl-amino]-1-déoxy-D-glucitol, le [hexadécyloxycarbonyl-méthyl-amino]-1-déoxy-D-glucitol, le 1-[octadécyloxycarbonyl-méthyl-amino]-1-déoxy-D-glucitol, le 1-[docosyloxy carbonyl-méthyl-amino]-1-déoxy-D-glucitol, et le 1-[tétracosanoyl-méthyl-amino]-1-déoxy-D-glucitol.

On peut citer encore les amides dérivés de mélanges d'acides gras ou les carbamates dérivés de mélange d'alcools gras; les acides ou alcools gras pouvant être obtenus par synthèse ou par extraction à partir de cires naturelles, végétales ou animales.

A titre d'exemple, l'octacosanol extrait des germes de riz ou de blé et vendu par la société NIPPON OILS est un mélange d'alcools en C₂₆-C₃₆. Des mélanges d'acides riches en C₂₂ et C₂₄ peuvent être obtenus à partir, entre autre, des cires de riz, de camauba ou d'abeilles.

Les procédés de préparation des dérivés de formule (I) ci-dessus sont bien connus de l'homme de métier. On peut, par exemple, préparer les amides répondant à la formule (I) par la méthode décrite par E.K Hildreth, Biochem J. 207 (1982) 363.

Selon cette méthode, on prépare dans une première phase un anhydride mixte en faisant réagir un acide de formule R¹COOH, où R¹ est défini comme précédemment, avec un halogèno formiate d'alkyle inférieur, de préférence d'éthyle, dans un solvant convenable en présence d'une base, par exemple de la pyridine, pour former une solution d'anhydride mixte.

Dans une deuxième phase on fait réagir la solution d'anhydride mixte obtenue avec une N-alkylamine de formule : 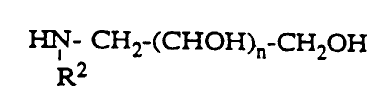 où R² et n sont définis comme précédemment, dans un solvant approprié, par exemple des alcanols, comme le méthanol, l'éthanol, ou encore le diméthylformamide.

On peut préparer les carbamates répondant à la formule (I) en faisant réagir une amine de formule 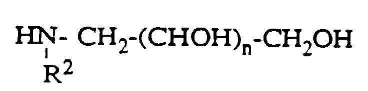 où R² et n sont définis comme précédemment avec un halogénoformiate d'alkyle en C₁₄-C₄₀, convenable, par exemple un chloroformiate, en présence d'hydrogénocarbonate de sodium, d'eau et d'un solvant convenable, par exemple le tétrahydrofurane.

La présente invention conceme également de nouveaux carbamates entrant dans le cadre de la formule générale (I) ci-dessus. Plus particulièrement, ces nouveaux carbamates répondent à la formule générale (Il) 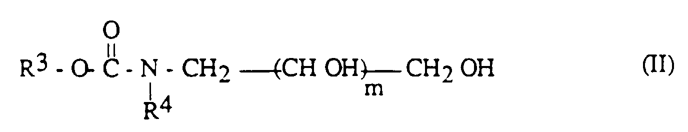 dans laquelle R³ est un radical aliphatique linéaire saturé en C₂₂-C₄₀, de préférence en C₂₂-C₂₆, R⁴ est un atome d'hydrogène ou un radical alkyle linéaire en C₁-C₆ et m est un entier de 1 à 5, de préférence égal à 4.
De préférence R³ est le radical docosyle et R⁴ est un radical méthyle.

Les compositions cosmétiques, en particulier les compositions pour le traitement des cheveux, de la peau, et pour l'hygiène bucco-dentaire conformes à l'invention sont caractérisées par le fait qu'elles contiennent au moins un dérivé répondant à la formule (I) ci-dessus dans un support cosmétiquement acceptable.

Lorsque les compositions selon l'invention sont utilisées pour le traitement des cheveux, on constate une amélioration du démêlage des cheveux mouillés et un apport de lissage et de douceur sur cheveux séchés.

Généralement, le ou les composés de formule (I) sont présents dans la composition en une concentration comprise entre 0,01 et 15% en poids par rapport au poids total de la composition et de préférence en une concentration de 0,1 à 10% en poids par rapport au poids total de la composition.

Le support cosmétiquement acceptable peut être constitué par une phase grasse ou par un milieu aqueux constitué uniquement par de l'eau ou par un mélange d'eau et d'un solvant cosmétiquement acceptable tel qu'un alcool inférieur en C₁-C₄ comme l'éthanol, l'isopropanol, le n-butanol; les alkylèneglycols, comme l'éthylèneglycol; les éthers de glycols.

Les compositions selon l'invention peuvent se présenter sous forme d'émulsion (lait, crème ou pommade), de dispersion vésiculaire, de stick solide, de solution ou de dispersions aqueuses éventuellement épaissies ou gélifiées, de spray ou de mousses aérosol.

Les compositions peuvent contenir en outre des agents tensio-actifs bien connus dans l'état de la technique tels que des agents tensio-actifs anioniques, non-ioniques, cationiques, amphotères ou zwitterioniques ou leurs mélanges.

Les compositions sont par exemple des lotions, des laits ou des crèmes émollients, des laits ou des crèmes pour les soins de la peau ou des cheveux, des crèmes, des lotions ou des laits démaquillants, des bases de fond de teint, des lotions, des laits ou des crèmes antisolaires, des lotions, des laits ou des crèmes de bronzage artificiel, des crèmes ou des mousses de rasage, des lotions après rasage, des shampooings, des après shampooings ou des mascaras.

Ces compositions peuvent également se présenter sous la forme de bâtons pour les lèvres destinés soit à les colorer, soit à éviter les gerçures, ou de produits de maquillage pour les yeux ou de fards et fonds de teint pour le visage.

Lorsque les compositions selon l'invention se présentent sous forme d'émulsions du type eau-dans-huile ou huile-dans l'eau, la phase grasse est essentiellement constituée d'un mélange de composé de formule (I) avec au moins une huile, et éventuellement un autre corps gras.

La phase grasse des émulsions peut constituer 5 à 60 % du poids total de l'émulsion.

La phase aqueuse desdites émulsions constitue de préférence 30 à 85 % du poids total de l'émulsion.

La proportion de l'agent émulsionnant peut être comprise entre 1 et 20 %, et de préférence entre 2 et 12 % du poids total de l'émulsion.

Lorsque les compositions selon l'invention se présentent sous forme de lotions huileuses, oléoalcooliques ou hydroalcooliques, elles peuvent constituer, par exemple, des lotions antisolaires contenant un filtre absorbant les rayons UV, des solutions adoucissantes pour la peau; les lotions huileuses peuvent en outre constituer des huiles moussantes contenant un tensio-actif oléosoluble, des huiles pour le bain, etc.

Parmi les principaux adjuvants pouvant être présents dans les compositions selon l'invention, on peut citer les corps gras tels que les huiles ou les cires minérales, animales ou végétales, les acides gras, les esters d'acides gras tels que les triglycérides d'acides gras ayant de 6 à 18 atomes de carbone, les alcools gras; les émulsionnants comme les alcools gras oxyéthylénés ou les alcoyléthers de polyglycérol, les solvants tels que les monoalcools ou polyalcools inférieurs contenant de 1 à 6 atomes de carbone ou encore l'eau.

Les mono- ou polyalcools plus particulièrement préférés sont choisis parmi l'éthanol, l'isopropanol, le propylèneglycol, le glycérol et le sorbitol.

A titre de corps gras, parmi les huiles minérales, on peut citer l'huile de vaseline; parmi les huiles animales, les huiles de baleine, de phoque, de menhaden, de foie de flétan. de morue, de thon, de tortue, de pied de boeuf, de pied de cheval, de pied de mouton, de vison, de loutre, de marmotte, etc.; parmi les huiles végétales, les huiles d'amande, de germe de blé, d'olive, de germe de maïs, de jojoba, de sésame, de toumesol, de palme, de noix, de karité, de shoréa, de macadamia, de pépins de cassis et similaires.

Parmi les esters d'acides gras, on peut utiliser des esters d'acides en C₁₂ à C₂₂ saturés ou insaturés et d'alcools inférieurs comme l'isopropanol ou le glycérol ou d'alcools gras en C₈ à C₂₂, linéaires ou ramifiés, saturés ou insaturés ou encore d'alcanediols-1,2 en C₁₀-C₂₂.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire microcristalline, la cire de Carnauba, le spermaceti, le beurre de cacao, le beurre de Karité, les cires de silicone, les huiles hydrogénées concrètes à 25°C, les sucroglycérides, les oléates, myristates, linoléates et stéarates de Ca, Mg et Al.

Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de polyglycérol, les alcools en C₁₂-C₁₈ comportant de 2 à 10 moles de glycérol.

Il peut aussi être utile d'utiliser des épaississants tels que les dérivés de cellulose, les dérivés d'acide polyacrylique, les gommes de guar ou de caroube ou la gomme de xanthane.

Les compositions conformes à l'invention peuvent se présenter sous forme de dispersion vésiculaire de lipides amphiphiles ioniques ou non-ioniques. Elles sont préparées notamment en faisant gonfler les lipides dans une solution aqueuse pour former des sphérules dispersées dans le milieu aqueux comme décrit dans l'article BANGHAM, STANDISH & WATKINS, J. Mol. Biol., 13,238 (1965) ou dans les brevets FR-2.315.991 et 2.416.008 de la demanderesse.

Les différents types de procédés de préparation sont décrits dans "Les liposomes en biologie cellulaire et pharmacologie", Edition INSERAM/John Liberry Eurotext, 1987, pages 6 à 18.

La composition selon l'invention peut également contenir des adjuvants habituellement utilisés en cosmétique et notamment des produits hydratants, des adoucissants, des produits pour le traitement d'affections cutanées, des filtres solaires, des germicides, des colorants, des conservateurs, des parfums et des propulseurs, des agents séquestrants.

Les exemples suivants sont destinés à illustrer l'invention.

### EXEMPLES DE PREPARATION

### Exemple 1

### Préparation du 1-[docosanoyl-méthyl-amino]-1-déoxy-D-glucitol

### Phase A: Préparation de l'anhydride mixte

Dans un réacteur, on introduit 140 ml de tétrahydrofurane et 23,4 g de chloroformiate d'éthyle, on ajoute régulièrement à 40°C une solution de 70 g d'acide béhenique vendu sous la dénomination "PRIFRAC 2989" par la société UNICHEMA en solution dans 140 ml de tétrahydrofurane puis on neutralise par 21,8 g de triéthylamine et on filtre la solution qui sera utilisée telle quelle à la phase B.

### Phase B:

Dans un réacteur, on dissout, à 60°C, 40,1 g de N-méthylglucamine dans 400 ml de diméthylformamide puis on ajoute lentement l'anhydride mixte obtenu à la phase A et on poursuit la réaction à 60°C pendant 3 heures.

En fin de réaction, on dilue le mélange réactionnel par 400 ml d'eau et on refroidit à 40°C. Le produit cristallisé, essoré, est recristallisé dans 1 l d'éthanol à 95°. On obtient 94,6 g d'un produit blanc, soit un rendement final de 88 %.

### ANALYSES

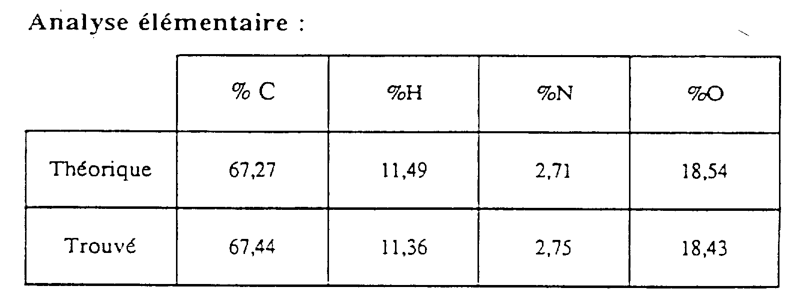

Point de fusion : 114°C

### Exemple 2

### Préparation du 1-[hexadécyloxycarbonyl-méthyl-amino]-1-déoxy-D-glucitol

Dans un réacteur, on dissout 11,7 g de N-méthylglucamine dans un mélange de 30 ml d'eau et de 40 ml de tétrahydrofurane, puis on ajoute et disperse 20 g d'hydrogénocarbonate de sodium.

En maintenant la température du mélange réactionnel à 20°C, on ajoute goutte à goutte 18,3 g de chloroformiate d'hexadécyle commercialisé par la S.N.P.E., puis on laisse pendant deux heures en réaction et on dilue avec 100 ml de tétrahydrofurane.

On filtre le mélange réactionnel ; le produit solide récupéré est solubilisé dans 500 ml d'acétone ; la fraction insoluble est éliminée par filtration à chaud.

Après refroidissement à 1°C pendant 12 heures, le produit cristallisé est récupéré et séché. On obtient 15,5 g d'un produit pur blanc, soit un rendement final de 56 %.

### ANALYSES:

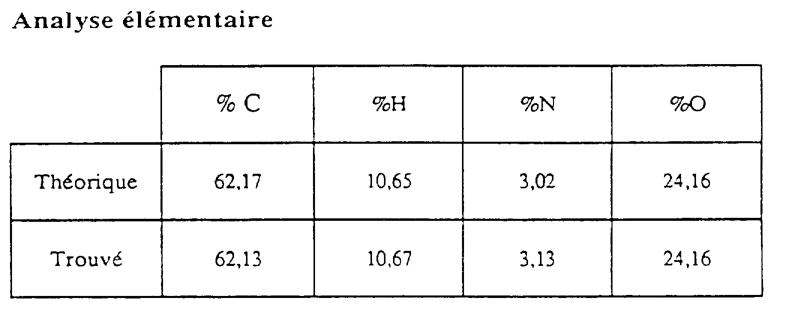

Point de fusion : 80°C

### Exemple 3

### Préparation du 1-[octadécyloxycarbonyl-méthyl-amino]-1-déoxy-glucitol

On répète le mode opératoire de l'exemple 2 en remplaçant le chloroformiate d'hexadécyle par du chloroformiate d'octadécyle. Le produit attendu obtenu est blanc et le rendement final est de 66 %.

### ANALYSES

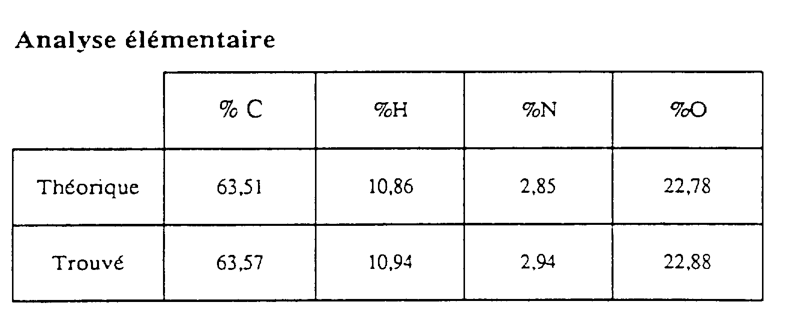

Point de fusion : 85°C

### Exemple 4

### Préparation du 1-[docosyl-oxycarbonyl-méthyl-amino]-1-déoxy-D-glucitol

Dans un réacteur, on dissout 70,2 g de N-méthyl glucamine dans un mélange de 180 ml d'eau et 240 ml de tétrahydrofurane puis on ajoute et disperse 12 g d'hydrogenocarbonate de sodium.

En maintenant la température du mélange réactionnel à 5°C, on ajoute goutte à goutte pendant deux heures 140,1 g de chloroformiate de docosyle, en solution dans 100 ml de tétrahydrofurane; le mélange réactionnel est encore dilué par 1 l de tétrahydrofurane et laissé deux heures en réaction.

On filtre le mélange réactionnel; après décantation, on recueille la phase organique et on concentre puis recristallise le résidu dans un litre d'un mélange de 8 volumes d'acétone et 2 volumes de méthanol. On obtient 70 g d'un produit pur blanc, soit un rendement final de 36%.

### ANALYSES

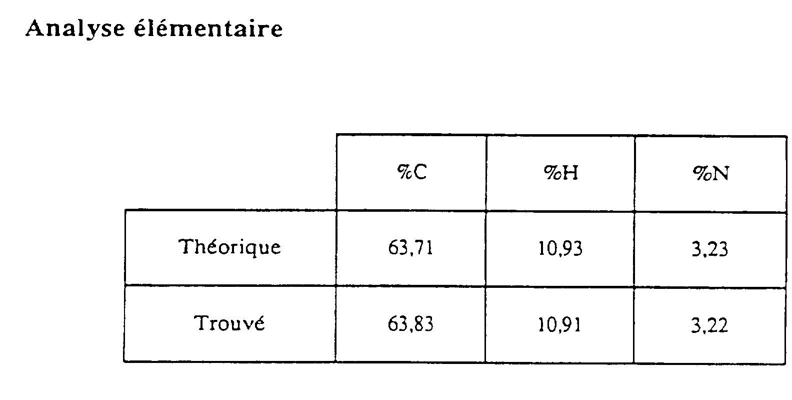

Point de fusion : 94,6°C.

### EXEMPLE 5

### Préparation du 1-[Docosanoylamino]-1-déoxy-D-glucitol

On répète le mode opératoire de l'exemple 1 en remplaçant la N-méthyl glucamine par 37,2 g de glucamine, on obtient 59 g du produit attendu, (R = 57 %), par recristallisation dans le propanol.

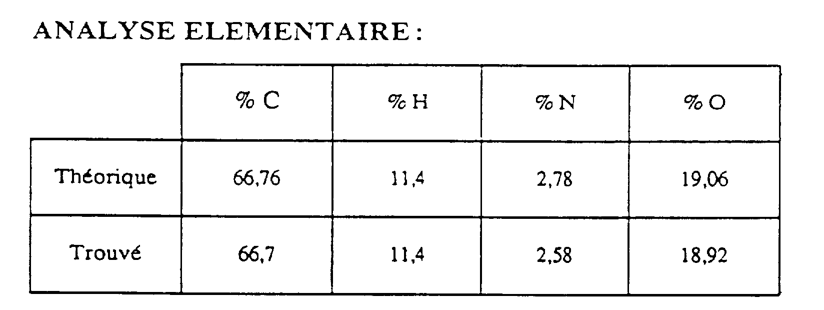

Point de fusion : 136°C

Les exemples suivant sont destinés à illustrer les formulations cosmétiques, selon la présente invention.

### EXEMPLE 6

### Préparation du 1-[tétracosanoyl-méthyl-amino]-1-déoxy-D-glucitol

### Phase A : préparation de l'anhydride mixte

Dans un réacteur, on introduit 1,55 g de chloroformiate d'éthyle en solution dans 10 ml de tétrahydrofurane.

A 0°C, on introduit lentement une solution de 5 g d'acide tétracosanoïque vendu sous la dénomination "NAFOL 24/26 Acid" par la Société CONDEA et de composition :
C₂₀:5 %,
C₂₂: 5 %,
C₂₄ : 60 %,
C₂₆: 22 %,
C₂₈ : 4 %,
dans 50 ml de tétrahydrofurane préalablement salifié par 1,44 g de triéthylamine. On laisse réagir pendant 2 heures, puis on filtre la solution qui sera utilisée telle qu'elle à la phase B.

### Phase B:

Dans un réacteur, on dissout, à 80° C, 2,6 g de N-méthyl glucamine dans 50 ml de diméthylformamide et on maintient la température à 60°C ; on ajoute ensuite lentement, l'anhydride mixte obtenu à la phase A et on poursuit la réaction à 60°C pendant 3 heures.
On refroidit à 4°C et on laisse cristalliser pendant 1 heure; après filtration, le solide est recristallisé dans du méthanol.
On obtient ainsi 3,2 g du produit attendu (R = 43 %)

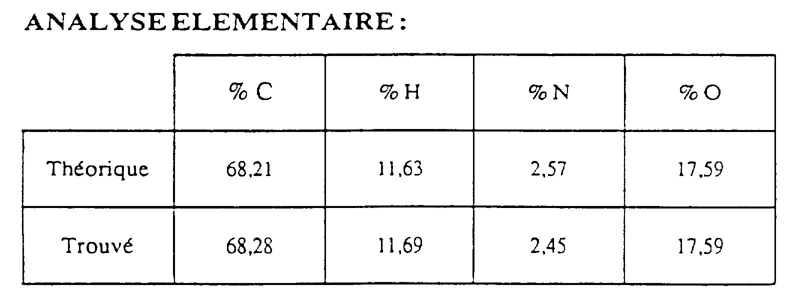

Point de fusion : 102°C
Le spectre de masse est conforme à la structure attendue.

### Exemple A: Shampooing

- Composé de l'exemple 1 5 g
- Alkyl (C₉/C₁₀/C₁₁ - 20/40/40) polyglucoside (1,4) vendu par la Société HENKEL à 500g % en matière active 15 g MA
- Conservateur q.s.
- Parfum q.s.
- Colorant q.s.
- Eau q.s.p. 100 g
pH ajusté à 7 avec de l'acide chlorhydrique

On obtient un liquide laiteux épaissi.

### EXEMPLE B : Shampooing

- Composé de l'exemple 1 0,1 g
- Lauryléthersulfate de sodium (C₁₂/C₁₄ - 70/30) 2.2 moles OE vendu sous la dénomination "EMPICOL ESB/3 FL" par la Société MARCHON à 28 % en matière active 15 g MA
- Cocoylbétaïne en solution aqueuse à 32 % 2.5 g MA
- Monoisopropanolamide d'acide de coprah vendu sous la dénomination "EMPILAN CIS" par la Société MARCHON 1 g
- Ether de cétyle et d'hydroxy 2 cétyl-stéaryle/alcool cétylique 2,5 g
- Parfum q.s.
- Conservateur q.s.
- Colorants q.s.
- Eau q.s.p. 100 g
pH ajusté à 6,7 avec de la triéthanolamine.

On obtient un liquide laiteux épaissi.

### EXEMPLE C : Shampooing

- Composé de l'exemple 1 1.5 g
- Dodécanediol polyglycérolé à 3.5 moles de glycérol 10 g MA
- Ethers d'hexadécanediol (3 moles) et de polyéthylène glycol (60 OE) 2.5 g
- Conservateurs q.s.
- Parfum q.s.
- Colorants q.s.
- Eau q.s.p. 100 g
pH ajusté à 7,6 avec de la triéthanolamine

On obtient un liquide épais translucide

### EXEMPLE D : Gel douche

- Composé de l'exemple 1 0,5 g
- Acide lauryl (C₁₂/C₁₄ - 70/30) éthercarboxylique 4,5 OE vendu sous la dénomination "AKYPO RLM 45" à 90 g % de matière active par la Société RIOIIN 10 g MA
- Cocoamido éthyl (N-hydroxyéthyl, N-carboxyméthyl)glycinate de sodium 5 g MA
- Lauroyl sarcosinate de sodium vendu sous la dénomination "ORAMIX L 30" par la Société SEPPIC à 30 g % MA 8 g MA
- Di-oléate de polyéthylène glycol (550E) et de propylène glycol/ eau (40/40/20) vendu sous la dénomination "ANTIL 141" liquide par la Société GOLDSCHMIDT à 40 g % MA 2 g MA
- Conservateur q.s.
- Parfum q.s.
- Colorant q.s.
- Eau q.s.p. 100 g
pH ajusté à 7,5 par de l'acide chlorhydrique.

On obtient un gel fluide limpide.

### EXEMPLE E : Après-shampooing

- Composé de l'exemple 1 2 g
- Chlorure de distéaryl diméthyl ammonium 2 g
- Hydroxyéthylcellulose vendu sous la dénomination de "NATROSOL 250 HHR" par la Société AQUALON 1 g
- Conservateur q.s.
- Parfum q.s.
- Colorant q.s.
- Eau q.s.p. 100 g
pH ajusté à 4,5 par de la triéthanolamine.

On obtient un gel fluide blanc.

### EXEMPLE F : Après-shampooing

- Composé de l'exemple 1 10 g
- Chlorure de distéaryl diméthylammonium 5 g
- Mélange d'alcools cétylstéarylique et de cétylstéarylique oxyéthyléné à 33 OE (80/20) 3 g
- Alcool cétylique 1 g
- Alcool stéarylique 1 g
- Conservateur q.s.
- Parfum q.s.
- Colorant q.s.
- Eau q.s.p. 100g
pH ajusté à 4 par de la triéthanolamine.

On obtient une crème épaisse blanche.

### EXEMPLE G : Emulsion Huile-dans eau - Soin après soleil

- 1-[docosanoyl-méthyl-amino]-1-déoxy-D-glucitol (composé de l'exemple 1) 0.5 g
- Glycérine 3 g
- Hydroxyde de sodium 0,24 g
- Alcool cétylique 2,5 g
- Acide éthylène diamine tétracétique (EDTA) 0,1 g
- Alcool stéarylique 1 g
- Sorbitol 3 g
- Huile de tournesol 6 g
- Monolaurate de sorbitane polyoxyéthyléné à 20 moles d'oxyde d'éthylène vendu sous la dénomination "TWEEN 20" par la Société ICI 2 g
- Acide polyacrylique réticulé vendu sous la dénomination "CARBOPOL 941" par la Société GOODRICH 0,3 g
   Extrait huileux de jaune d'oeuf 2 g
- Huile de silicone vendu sous la dénomination "SILBIONE ANTIMOUSSE 70 452" par la Société RHONE POULENC 0,15 g
- Cire d'abeille 1 g
- Farine d'avoine 0,5 g
- Beurre de karité 1.5 g
- Conservateurs, antioxydant, parfum q.s.
- Eau q.s.p. 100g

L'émulsion est réalisée selon le mode opératoire suivant :
1) Faire gonfler le "CARBOPOL 941" avec la glycérine dans une partie d'eau, neutraliser à pH 7 avec la soude, à 60°C sous agitation (Phase 1)
2) Ajouter dans la phase 1, les émulsionnants TWEEN 20 et extrait huileux de jaune d'oeuf, la farine d'avoine (Phase 2)
3) Dissoudre l'EDTA dans une partie d'eau à 60°C.

Ajouter le sorbitol, le beurre de karité et la cire d'abeille et l'huile de silicone, sous agitation à 60°C.

On laisse agiter 30 minutes (Phase 3)
4) Ajouter la phase 2 à la phase 3 à 60°C, sous agitation (Phase 4)
5) Faire fondre à 70°C l'huile de tournesol, les co-émulsionnants alcool cétylique, alcool stéarylique, les conservateurs de la phase grasse et composé de l'exemple 1 (Phase 5).
6) Sous agitation, mélanger les phases 4 et 5.
7) Laisser refroidir jusqu'à 40°C

Ajouter les conservateurs de la phase aqueuse et le parfum. Continuer l'agitation jusqu'à complet refroidissement.

### EXEMPLE H : Coloration directe

- Composé de l'exemple 2 0,1 g
- Monométhyléther de propylène glycol 10 g
- Nonylphénol oxyéthyléné (9 OE) commercialisé sous la dénomination "RHODIASURF NP 9 OR" par la Société RHONE POULENC 8g
- Diéthanolamide de coprah a été vendu sous la dénomination "COMPERLAN KD" par la société HENKEL 2 g
- N1, N4,N4-tris(β-hydroxyéthyl) 1,4-diamino 2-nitrobenzène 0.5 g
- 1-amino-2-nitro-4-B-hydroxyéthylamino-benzène 0.05 g
- Agent de pH q.s.p. pH 9
- Eau déminéralisée q.s.p. 100 g

### Mode d'application :

Les mèches de cheveux gris à 90 % de blancs, permanentés, sont immergées dans la composition colorante, à raison de 20 g de formule pour 3 g de cheveux. On laisse agir 30 minutes. Les cheveux sont ensuite rincés et séchés. Les cheveux sont colorés en violet.

### EXEMPLE I : Coloration d'oxydation alcaline - support crème

- Composé de l'exemple 1 0,3 g
- Alcool cétylstéarylique (C₁₆/C₁₈ 50/50) vendu sous la dénomination CIRE DE LANETTE O par la Société HENKEL 18 g
- 2-octyl dodécanol 3 g
- Alcool cétylstéarylique (C₁₆/C₁₈ 35/65) oxyéthyléné (15 OE) vendu sous la dénomination "MERGITAL CS 15" par la Société SINNOVA-HENKEL 3 g
- Laurylsulfate d'ammonium à 30 % MA 12 g en l'état
- Polymère de formule 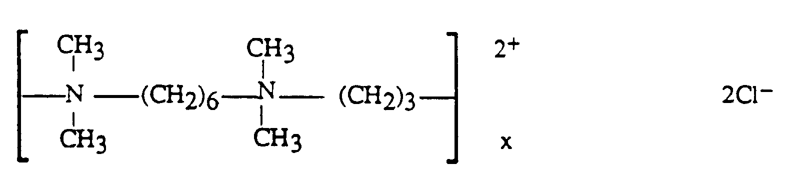 en solution aqueuse à 60 % de matière active vendu sous la dénomination "MEXOMER PO" par la Société CHIMEX 3 g tel que
- Thiolactate d'ammonium (à 50 % en équivalent d'acide thiolactique) 0,8 g
- Ammoniaque à 20 % de NH3 12 g
- Paraaminophénol 0,436 g
- 1-méthyl-2-hydroxy-4-β-hydroxyéthylaminobenzène 0,668 g
- Eau déminéralisée q.s.p. 100 g

### Mode d'application

La composition obtenue est diluée extemporanément avec 1,5 fois son poids d'eau oxygénée à 20 volumes, dont le pH est 3.

Le mélange ainsi réalisé est appliqué sur des cheveux gris à 90 % de blancs, permanentés ou non, à raison de 28 g pour 3 g de cheveux. On laisse agir 30 minutes.

Les cheveux sont ensuite rincés, lavés avec un shampooing et rincés. Les cheveux sont colorés en cuivré ou en cuivré intense suivant qu'il s'agit respectivement de cheveux naturels ou permanentés.

### EXEMPLE J : Emulsion huile-dans-eau antisolaire

- Composé de l'exemple 2 2 g
- Acide stéarique 2 g
- Alcool stéarylique 1 g
- Huile de vaseline 10 g
- Pararnéthoxy cinnamate de 2-éthylhexyle vendu sous la dénomination "PARSOL MCX" par la Société GIVAUDAN 5 g
- Glycérine 3 g
- Sorbitol 2 g
- Copolymère réticulé acide acrylique/acrylate d'alkyle C₁₀-C₃₀ vendu sous la dénomination "PEMULEN TR-1" par la Société GOODRICH 0,3 g
- Triéthanolamine 0,4 g
- Conservateurs q.s.
- Eau q.s.p. 100 g

### EXEMPLE K: Emulsion huile-dans-eau antisolaire

- Composé de l'exemple 4 2 g
- Acide stéarique 2 g
- Alcool stéarylique 1 g
- Huile de vaseline 12,5 g
- Paraméthoxycinnamate de 2-éthylhexyle vendu sous la dénomination "PARSOL MCX" par la Société GIVAUDAN 2.5 g
- Glycérine 3 g
- Copolymère réticulé acide acrylique/acrylate d'éthyle C₁₀-C₃₀ vendu sous la dénomination "PEMULEN TR-1" par la Société GOODRICH 0.3 g
- Triéthanolamine 0,4 g
- Conservateurs q.s.
- Eau q.s.p. 100g

### EXEMPLE L : Après-shampooing

- Composé de l'exemple 6 0.3 g
- Chlorure de distéaryldiméthyl ammonium 2 g
- Parfum - colorant q.s.
- Eau q.s.p. 100 g
pH ajusté à 5 par la soude

### EXEMPLE M : Shampooing

- Composé de l'exemple 4 3 g
- Alkyl (C₉/C₁₀/C₁₁ - 20/40/40)polyglucoside (1.4) vendu par la Société HENKEL à 50 g % en Matière Active 12 g MA
- Conservateur, parfum, colorant q.s.
- Eau q.s.p. 100 g
pH ajusté à 6 avec de l'acide chlorhydrique.

On obtient une composition légèrement opalescente.

### EXEMPLE N : Stick déodorant

- Composé de l'exemple 4 0,2 g
- Hydroxyde de sodium 1 g
- Acide stéarique 5 g
- Propylène glycol 50 g
- 2,4,4'-trichloro 2 hydroxy diphényléther vendu sous la dénomination "IRGASAN DP 300" par la Société CIBA-GEIGY 0,3 g
- Eau q.s.p. 100 g

### EXEMPLE O: Lotion de coiffage

- Copolymère vinylpyrrolidone-acétage de vinyle (65/35) vendu sous la dénomination "RESINE PVP/VA S 630 L" par la Société GAF 10 g
- Composé de l'exemple 2 0,25 g
- Poly(hydroxypropyléther) préparé par condensation en catalyse alcaline de 3,5 moles de glycidol sur un mélange d'alpha-diols ayant 10 à 14 carbones 0,1 g
- Alcool éthylique 45,2 g
- Parfum, colorant, conservateur q.s.
- Eau déminéralisée q.s.p. 100 g
pH spontané à 4,9.

On obtient une solution opalescente.

### EXEMPLE P: mousse de coiffage

On prépare une mousse de coiffage aérosol ayant la composition suivante :
- Copolymère hydroxyéthyl cellulose - chlorure de diallyldiméthyl ammonium "CELQUAT LOR" par la Société NATIONAL STARCH 1 g
- Composé de l'exemple 6 0,2 g
- Alcool éthylique 17,3 g
- Octylphénol oxyéthyléné à 10 moles d'oxyde d'éthylène vendu sous la dénomination "SCUROL O" par la Société SFOS 0,2 g
- Parfum, colorant, conservateur q.s.
- Eau q.s.p. 100g

On introduit 90 g de la composition obtenue dans un boîtier aérosol sous tube plongeur. On procède à la fixation de la valve et à la fermeture hermétique du récipient, puis on introduit 10 g d'un mélange propulseur butane/isobutane/propane (0,32 MPa (3,2 bars)).

### EXEMPLE Q

On prépare une composition réductrice de déformation permanente des cheveux en procédant au mélange des ingrédients suivants :

### Composition réductrice :

- Composé de l'exemple 2 0,25 g
- Octyl phénol oxyéthyléné à 10 moles d'oxyde d'éthylène vendu sous la dénomination "SCUROL O" par la Société SFOS 2 g
- Eau déminéralisée chaude 60 g
- Acide thioglycolique 9 g
- Ammoniaque à 20 % q.s. pH 8.2
- Eau déminéralisée q.s.p. 100 g

Cette composition est appliquée sur des cheveux mouillés préalablement enroulés sur des rouleaux. Après avoir laissé agir la composition 15 minutes, on rince abondamment à l'eau puis on applique la composition suivante :

### Composition oxydante:

- Eau oxygénée 1,5 g
- Lauryléther sulfate de sodium oxyéthyléné avec 2 moles d'oxyde d'éthylène 3,75 g
- Acide citrique 0,5 g
- Hydrogénophosphate de sodium 0.5 g
- Parfum 0,3 g
- Eau déminéralisée q.s.p. 100 g

On laisse agir la composition oxydante pendant environ 15 minutes, puis on enlève les rouleaux et rince abondamment la chevelure à l'eau. Après séchage sous casque, les cheveux présentent de belles boucles.

### EXEMPLE R

Selon le même mode de réalisation de l'exemple Q, on procède à une déformation pemanente des cheveux à l'aide de la composition réductrice suivante :
- Composé de l'exemple 2 0,25 g
- Octyl phénol oxyéthyléné à 10 moles d'oxyde d'éthylène vendu sous la dénomination "SCUROL O" par la Société SFOS 2 g
- Eau déminéralisée chaude 20 g
- Thioglycolate de glycérol (70 % matière active dans le glycérol) 10 g
- Triéthanolmaine q.s. pH 7
- Eau déminéralisée q.s.p. 100 g

La composition oxydante est la même que celle de l'exemple Q.

### EXEMPLE S: Crème de soin eau-dans-huile teintée

- Alkyldiméthicone copolyol vendu sous la dénomination "ABIL EM 90" par la Société GOLDSCHMIDT 5,00 g
- Stéaralkonium hectorite 2,00 g
- Octyldodécanol 8, 00 g
- Décaméthylcyclopentasiloxane 20,00 g
- Parfum q.s.
- Conservateur q.s.
- Glycérine 3,00 g
- Pigments 7,00 g
- Composé de l'exemple 1 0,50 g
- Chlorure de sodium 1.50 g
- Eau déminéralisée q.s.p. 100 g

### EXEMPLE T: Crème de soin eau-dans-huile teintée

- Silicate de magnésium et d'aluminium 0,50 g
- Carboxyméthyl cellulose 0,15 g
- Propylène glycol 5,00 g
- Conservateurs q.s.
- Composé de l'exemple 4 1,00 g
- Alcool de lanoline 1,50 g
- Stéarate de glycéryle 1.00 g
- Acide stérarique 2,50 g
- Triéthanolamine 1,50 g
- Triglycérides d'acide caprique/caprylique 6,00 g
- Polyisobutène 10,00 g
- Poudre de nylon 3,00 g
- Pigments 10,00 g
- Eau déminéralisée q.s.p. 100 g

### EXEMPLE U : Mascara

- Stéarate de triéthanolamine 15,00 g
- Paraffine 3,00 g
- Cire d'abeille 8,00 g
- Composé de l'exemple 1 0.50 g
- Colophane 2,00 g
- Ozokérite 10,00 g
- Conservateurs q.s.
- Gomme arabique 0,60 g
- Hydrolysat de kératine 1,00 g
- Pigments 6,00 g
- Eau déminéralisée q.s.p. 100 g

### EXEMPLE V: Base traitante pour ongle :

- Nitrocellulose 12,00 g
- Résine toluène sulfonamide-formaldéhyde 9,00 g
- Camphre 1,00 g
- Phtalate de dibutyle 6,05 g
- Acétate de butyle 24,00 g
- Acétate isopropylique 6,00 g
- Stéaralkonium hectorite 1,00 g
- Composé de l'exemple 4 0,01 g
- Acide citrique 0.02 g
- Toluène q.s.p. 100 g

### EXEMPLE W: Rouge à lèvres

- Butylhydroxytoluène 0,20 g
- Lanoline liquide 17,50 g
- Cire microscristalline 15,00 g
- Triglycérides d'acides caprique/caprylique 11,00 g
- Béhénate d'oxtyl glycéryl 11,00 g
- Pigments 3,00 g
- Micatitane 6,00 g
- Parfum 0,50 g
- Huile de ricin q.s.p. 100 g

### EXEMPLE X: Rouge à lèvres

- Parfum 0,50 g
- Triglycérides d'acides caprique/caprylique 9,10 g
- Huile de ricin 9,10 g
- Butylhydroxytoluène 0,16 g
- Lanoline liquide 12.70 g
- Lanoline isopropyle 4,50 g
- Composé de l'exemple 1 0,50 g
- Cire microcristalline 11,00 g
- Copolymère acétate de vinyle/stéarate d'allyle (65/35) 4,50 g
- Béhenate d'octyl glycéryle 9,10 g
- Pigments 9,00 g
- Huile de sésame q.s.p. 100 g

### EXEMPLE Y : Après-shampooing

- Chlorure de distéaryl diméthyl ammonium 2,00 g
- Composé de l'exemple 5 0,5 g
- N-(hydroxyméthyl)-N-(1,3-dihydroxyméthyl-2,5-dioxo-4-imidazolidinyl)-N'-(hydroxyméthyl) urée 0,1 g
- Eau q.s.p. 100 g
pH spontané : 4

## Revendications

1. Utilisation comme agent de conditionnement dans une compositions cosmétiques d'un ou plusieurs dérivés lipophiles des amino déoxyalditols répondant à la formule générale : 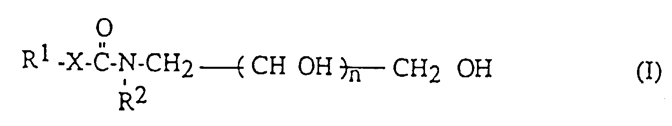 dans laquelle :
- R¹ est un radical aliphatique linéaire saturé en C₁₄-C₄₀;
- R² est un atome d'hydrogène ou un radical alkyle linéaire en C₁-C₆;
- X est un atome d'oxygène ou un radical méthylène; et
- n est un entier de 1 à 5;
sous réserve que lorsque X est un radical méthylène, R¹ comporte de 21 à 39 atomes de carbone.

2. Utilisation selon la revendication 1, caractérisée par le fait que R¹ est un radical aliphatique linéaire saturé en C₁₄-C₃₂ lorsque X représente un atome d'oxygène.

3. Utilisation selon la revendication 1, caractérisée par le fait que R¹ est un radical aliphatique linéaire en C₂₁-C₂₉ et X est un radical méthylène.

4. Utilisation selon l'une quelconque des revendications précédentes, caractérisée par le fait que R² est un radical méthyle.

5. Composition cosmétique, caractérisée par le fait qu'elle contient dans un milieu cosmétiquement acceptable au moins un dérivé lipophile d'amino déoxyalditol répondant à la formule générale : 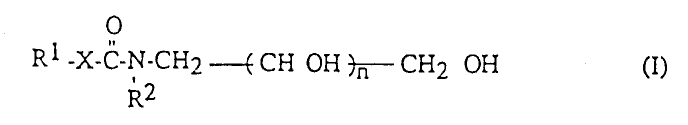 dans laquelle
- R¹ est un radical aliphatique linéaire saturé en C₁₄-C₄₀;
- R² est un atome d'hydrogène ou un radical alkyle linéaire en C₁-C₆;
- X est un atome d'oxygène ou un radical méthylène; et
- n est un entier de 1 à 5,
sous réserve que lorsque X est un radical méthylène, R¹ comporte de 21 à 39 atomes de carbone.

6. Composition selon la revendication 5, caractérisée par le fait que R¹ est un radical aliphatique linéaire saturé en C₁₄-C₃₂ et X est un atome d'oxygène.

7. Composition selon la revendication 5, caractérisée par le fait que R¹ est un radical aliphatique linéaire saturé en C₂₁-C₂₉ et X est un radical méthylène.

8. Composition selon l'une quelconque des revendications 5 à 7, caractérisée par le fait que R² est un radical méthyle.

9. Composition selon l'une quelconque des revendications 5 à 8, caractérisée par le fait que le ou les dérivés de formule (I) sont présents en une concentration comprise entre 0,01 et 15 % en poids par rapport au poids total de la composition, et de préférence entre 0,1 et 10 %.

10. Composition selon l'une quelconque des revendications 5 à 9, contenant des adjuvants choisis parmi les huiles, les cires, les émulsionnants, les solvants, les épaississants, les hydratants, les adoucissants, les filtres solaires, les germicides, les colorants, les conservateurs, les parfums, les propulseurs et les agents séquestrants.

11. Composition selon l'une quelconque des revendications 5 à 10 se présentant sous la forme d'émulsion, de dispersion vésiculaire, de stick solide, de solution ou de dispersion aqueuse, de spray ou de mousse aérosol.

12. Composition selon l'une quelconque des revendications 5 à 10 se présentant sous la forme de lotion, de lait ou de crème pour les soins des cheveux, de shampooing, et d'après-shampooing.

13. Composé caractérisé en ce qu'il répond à la formule générale : 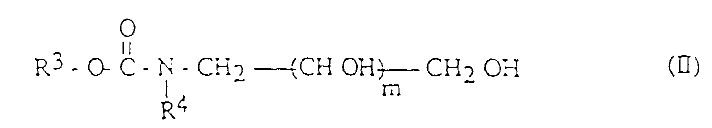 dans laquelle R³ est un radical aliphatique linéaire saturé en C₂₂-C₄₀, de préférence en C₂₂-C₂₆, R⁴ est un atome d'hydrogène ou un radical alkyle linéaire en C₁-C₆ et m est un entier de 1 à 5, de préférence égal à 4.

14. 1-[docosyl-oxycarbonyl-méthyl-amino]-1-déoxy-D-glucitol.

## Patentansprüche

1. Als Konditioniermittel in einer kosmetischen Zusammensetzung vorgesehene Verwendung eines oder mehrerer lipophiler Derivate von Aminodeoxyalditen der allgemeinen Formel (I) : 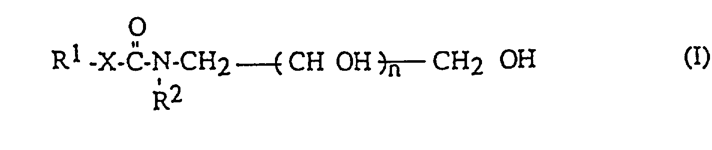 worin gilt:
R¹ ist ein gesättigter linearer aliphatischer C₁₄₋₂₀-Rest;
R² ist ein Wasserstoffatom oder ein linearer C₁₋₆-Alkylrest;
X ist ein Sauerstoffatom oder ein Methylenrest; und
n ist eine ganze Zahl von 1 bis 5,
mit der Maßgabe, daß, wenn X ein Methylenrest ist, R¹ 21 bis 39 Kohlenstoffatome aufweist.

2. Verwendung gemäß Anspruch 1,
dadurch **gekennzeichnet**, daß
R¹ ein gesättigter linearer C₁₄₋₃₂-Alkylrest ist, wenn X ein Sauerstoffatom darstellt.

3. Verwendung gemäß Anspruch 1,
dadurch **gekennzeichnet**, daß
R¹ ein linearer aliphatischer C₂₁₋₂₉-Rest und X ein Methylenrest sind.

4. Verwendung gemäß einem der vorhergehenden Ansprüche,
dadurch **gekennzeichnet**, daß
R² ein Methylrest ist.

5. Kosmetische Zusammensetzung,
dadurch **gekennzeichnet**, daß
sie in einem kosmetisch geeigneten Milieu mindestens ein lipophiles Aminodeoxyaldit-Derivat der allgemeinen Formel enthält: 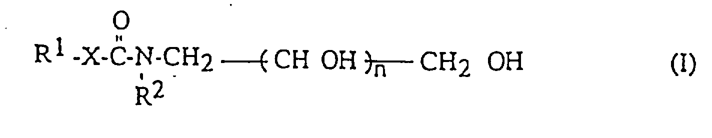 worin gilt:
R¹ ist ein gesättigter linearer aliphatischer C₁₄₋₂₀-Rest;
R² ist ein Wasserstoffatom oder ein linearer C₁₋₆-Alkylrest;
X ist ein Sauerstoffatom oder ein Methylenrest; und
n ist eine ganze Zahl von 1 bis 5,
mit der Maßgabe, daß, wenn X ein Methylenrest ist, R¹ 21 bis 39 Kohlenstoffatome aufweist.

6. Zusammensetzung gemäß Ansrpuch 5,
dadurch **gekennzeichnet**, daß
R¹ ein gesättigter linearer aliphatischer C₁₄₋₃₂-Rest und X ein Sauerstoffatom sind.

7. Zusammensetzung gemäß Anspruch 5,
dadurch **gekennzeichnet**, daß
R¹ ein gesättigter linearer aliphatischer C₂₁₋₂₉-Rest und X ein Methylenrest sind.

8. Zusammensetzung gemäß einem der Ansprüche 5 bis 7,
dadurch **gekennzeichnet**, daß
R² ein Methylrest sind.

9. Zusammensetzung gemäß einem der Ansprüche 5 bis 8,
dadurch **gekennzeichnet**, daß
das oder die Derivate der Formel (I) in einer Konzentration von 0,01 bis 15 und vorzugsweise von 0,1 bis 10 Gew.% vorhanden sind, bezogen auf das Gesamtgewicht der Zusammensetzung.

10. Zusammensetzung gemäß einem der Ansprüche 5 bis 9, enthaltend Hilfsstoffe, ausgewählt aus Ölen, Wachsen, Emulgatoren, Lösungsmitteln, Verdickungsmitteln, Hydratisiermitteln, weichmachenden Mitteln, Sonnenfilterstoffen, Germiziden, Farbstoffen, Konservierungsitteln, Parfüm-Produkten, Treibmitteln und aus Sequestriermitteln.

11. Zusammensetzung gemäß einem der Ansprüche 5 bis 10, die in Form einer Emulsion, bläschenartigenDispersion, eines Feststoff-Stifts, einer wässrigen Lösung oder dispersion, eines Spray oder Aerosolschaums vorliegt.

12. Zusammensetzung gemäß einem der Ansprüche 5 bis 10, die in Form einer Lotion, Milch oder Creme zur Pflege der Haare, eines Shampoo und Nach-Shampoo vorliegt.

13. Verbindung,
dadurch **gekennzeichnet**, daß
sie die allgemeine Formel aufweist: 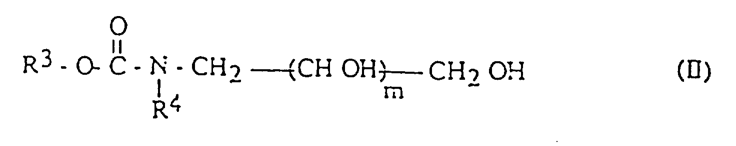 worin R³ ein gesättigter linearer aliphatischer C₂₂₋₄₀- und vorzugsweise ein C₂₂₋₂₆-Rest, R⁴ ein Wasserstoffatom oder ein linearer C₁₋₆-Alkylrest und m eine ganze Zahl von 1 bis 5 und vorzugsweise gleich 4 sind.

14. 1-[Docosyloxycarbonyl-methyl-amino]-1-deoxy-D-glucit.

## Claims

1. Use, as a conditioning agent in a cosmetic compositions, of one or more lipophilic derivatives of amino deoxyalditols corresponding to the general formula: 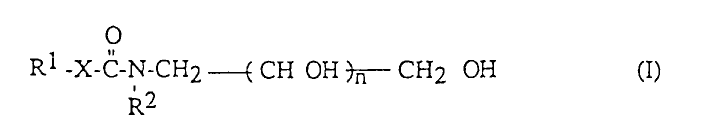 in which:
- R¹ is a saturated linear C₁₄-C₄₀ aliphatic radical;
- R² is a hydrogen atom or a linear C₁-C₆ alkyl radical;
- X is an oxygen atom or a methylene radical; and
- n is an integer from 1 to 5;
with the proviso that when X is a methylene radical R¹ contains from 21 to 39 carbon atoms.

2. Use according to Claim 1, characterized in that R¹ is a saturated linear C₁₄-C₃₂ aliphatic radical when X represents an oxygen atom.

3. Use according to Claim 1, characterized in that R¹ is a linear C₂₁-C₂₉ aliphatic radical and X is a methylene radical.

4. Use according to any one of the preceding claims, characterized in that R² is a methyl radical.

5. Cosmetic composition, characterized in that it contains, in a cosmetically acceptable medium, at least one lipophilic derivative of amino deoxyalditol corresponding to the general formula: 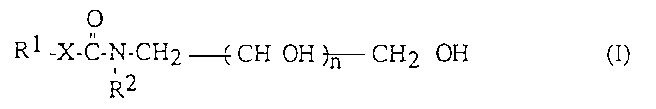 in which:
- R¹ is a saturated linear C₁₄-C₄₀ aliphatic radical;
- R² is a hydrogen atom or a linear C₁-C₆ alkyl radical;
- X is an oxygen atom or a methylene radical; and
- n is an integer from 1 to 5;
with the proviso that when X is a methylene radical R¹ contains from 21 to 39 carbon atoms.

6. Composition according to Claim 5, characterized in that R¹ is a saturated linear C₁₄-C₃₂ aliphatic radical and X is an oxygen atom.

7. Composition according to Claim 5, characterized in that R¹ is a linear C₂₁-C₂₉ aliphatic radical and X is a methylene radical.

8. Composition according to any one of Claims 5 to 7, characterized in that R² is a methyl radical.

9. Composition according to any one of Claims 5 to 8, characterized in that the derivative or derivatives of formula (I) are present at a concentration between 0.01 and 15% by weight relative to the total weight of the composition, and preferably between 0.1 and 10%.

10. Composition according to any one of Claims 5 to 9, which contains adjuvants chosen from oils, waxes, emulsifying agents, solvents, thickening agents, moisturizing agents, emollients, sunscreen agents, germicides, dyes, preserving agents, fragrances, propellants and sequestering agents.

11. Composition according to any one of Claims 5 to 10, which is provided in the form of an emulsion, a vesicle dispersion, a solid stick, an aqueous dispersion or solution, an aerosol foam or spray.

12. Composition according to any one of Claims 5 to 10, which is provided in the form of a lotion, a milk or a cream for hair care, a shampoo and a conditioner.

13. Compound, characterized in that it corresponds to the general formula: 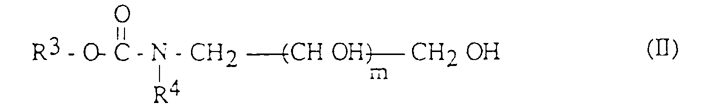 in which R³ is a saturated linear C₂₂-C₄₀, preferably a C₂₂-C₂₆, aliphatic radical, R⁴ is a hydrogen atom or a linear C₁-C₆ alkyl radical and m is an integer from 1 to 5, preferably equal to 4.

14. 1-[Docosyl-oxycarbonyl-methyl-amino]-1-deoxy-D-glucitol.
